# EUROPÄISCHE PATENTSCHRIFT

(11) **EP 2 241 550 B1**
(45) Veröffentlichungstag und Bekanntmachung des Hinweises auf die Patenterteilung: **26.07.2017**
(21) Anmeldenummer: 10003663.1
(22) Anmeldetag: 01.04.2010
(51) Int. Cl.: C07C 68/02, C07C 69/96, C01D 3/04

(54) **Verfahren zur Herstellung von Diarylcarbonat**
Method for manufacturing diaryl carbonate
Procédé de fabrication de carbonates de diaryle

(30) Priorität: 17.04.2009 DE 102009017862
(43) Veröffentlichungstag der Anmeldung: 20.10.2010
(73) Patentinhaber: Covestro Deutschland AG, 51373 Leverkusen (DE)
(72) Erfinder: Ooms, Pieter, Dr., 47800 Krefeld (DE); Bulan, Andreas, 40764 Langenfeld (DE); Rechner, Johann, Dr., 47906 Kempen (DE); Weber, Rainer, Dr., 51519 Odenthal (DE); Traving, Michael, Dr., 51399 Burscheid (DE); Buts, Marc, 2570 Duffel (BE); Vanden Eynde, Johan, 9052 Zwijnaarde (BE)
(74) Vertreter: Levpat

(56) Entgegenhaltungen:
- EP-A2- 1 216 982
- EP-A2- 1 894 914
- WO-A1-01/38419

## Beschreibung

Die Erfindung betrifft ein kombiniertes Verfahren zur Herstellung von Diarylcarbonat und Aufkonzentrierung von Natriumchlorid-haltigem Prozessabwasser durch osmotische Destillation unter gleichzeitiger Verdünnung der aus der Elektrolyse erhaltenen Natronlauge für das Diphenylcarbonat-Herstellungsverfahren.

Die Herstellung von Diarylcarbonaten (Diphenylcarbonat) erfolgt üblicherweise durch ein kontinuierliches Verfahren, durch Herstellung von Phosgen und anschließende Reaktion von Monophenolen und Phosgen in einem inerten Lösungsmittel in Gegenwart von Alkali und einem Stickstoffkatalysator in der Grenzfläche.

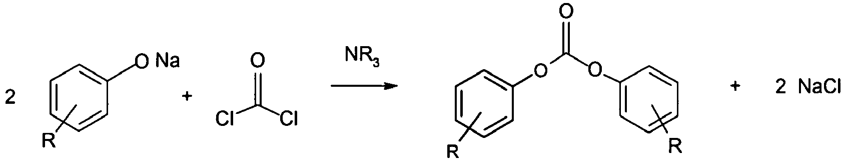

Die Herstellung von Diarylcarbonaten z.B. durch den Phasengrenzflächenprozess ist prinzipiell in der Literatur beschrieben, siehe z.B. in Chemistry and Physics of Polycarbonates, Polymer Reviews, H. Schnell, Vol. 9, John Wiley and Sons, Inc. (1964), S. 50/51.

In US-A-4 016 190 wird ein Herstellungsverfahren von Diarylcarbonaten beschrieben, das bei Temperaturen >65°C betrieben wird. Der pH-Wert wird bei diesem Verfahren zunächst niedrig (pH 8 bis 9) und anschließend hoch (10 bis 11) eingestellt.

Optimierungen des Verfahrens durch Verbesserung der Durchmischung und Einhaltung eines engen Temperatur- und pH-Profils als auch Isolierung des Produkts werden in EP1219589 A1, EP1216981 A2, EP1216982 A2 und EP784048 A1 beschrieben.

Bei diesen bekannten Verfahren macht jedoch ein hoher Restphenolwert im Abwasser dieser Prozesse, welche die Umwelt belasten können und die Klärwerke vor eine erhöhte Abwasserproblematik stellen, aufwendige Reinigungsoperationen erforderlich. So beschreibt WO 03/070639 A1 eine Entfernung der organischen Verunreinigungen im Abwasser durch eine Extraktion mit Methylenchlorid.

Üblicherweise wird die Natriumchlorid-haltige Lösung von Lösungsmitteln und organischen Resten befreit und wird dann entsorgt.

Gemäß EP 1200359 B1 (WO2000078682 A1) oder US-A-6340736 können die Natriumchlorid-haltigen Abwässer durch Ozonolyse gereinigt und dann bei der Natriumchlorid-Elektrolyse eingesetzt werden. Nachteil dieses Verfahren ist die sehr kostenintensive Ozonolyse.

Gemäß EP 541114 A2 wird ein Natriumchlorid-haltiger Abwasserstrom bis zur vollständigen Entfernung des Wassers eingedampft und das zurückbleibende Salz mit den organischen Verunreinigungen einer thermischen Behandlung unterzogen, wodurch die organischen Bestandteile zersetzt werden. Besonders bevorzugt ist dabei der Einsatz von Infrarotstrahlung. Nachteil des Verfahrens ist, dass das Wasser vollständig verdampft werden muss, so dass das Verfahren wirtschaftlich nicht durchführbar ist.

Gemäß WO 03/70639 A1 wird das Abwasser aus einer DPC-Produktion durch Extraktion gereinigt und dann der Natriumchlorid-Elektrolyse zugeführt. Es können jedoch nur max. 26 % des Natrium-chlorids aus dem Abwasser der DPC-Produktion in die NaCl-Elektrolyse zurückgeführt werden, da bei größeren Mengen an NaCl-haltigem Abwasser das mit dem NaCl-haltigen Abwasser in die Elektrolyse eingebrachte Wasser die Wasserbilanz der Natriumchlorid-Elektrolyse aus dem Gleichgewicht bringen würde.

Die Natriumchlorid-haltigen Lösungen, die bei der DPC-Produktion anfallen, haben typischerweise einen Natriumchlorid-Gehalt von 13 bis 17 Gew.-%. Somit kann nie das in den Lösungen vorhandene gesamte Natriumchlorid wieder bei der NaCl-Elektrolyse zu Chlor und Natronlauge recycelt werden. Bei einer Natriumchlorid-Konzentration von 17 Gew.-% gelingt bei der Standard-Natriumchlorid-Elektrolyse mit einer handelsüblichen Ionenaustauschermembran, die einen Wassertransport von 3,5 mol Wasser je mol Natrium aufzeigt, nur der Einsatz von ca. 23 % des Natriumchlorids aus dem Natriumchlorid-haltigen Lösungen. Bei Aufkonzentrierung bis zu einer gesättigten Natriumchlorid-Lösung von ca. 25 Gew.-% gelänge eine Recyclingquote von 38 % des in der Natriumchlorid-haltigen Lösung enthaltenden Natrium-chlorids. Ein vollständiges Recycling der Natriumchlorid-haltigen Lösung ist derzeit nicht bekannt.

EP 1 894 914 A2 beschreibt ein Verfahren zur Verstellung von Diarylcarbonate und Verarbeitung mindestens eines Teils der dabei anfallenden Alkalichlorid-haltigen Lösung in einer nachgeschalteten Alkalichlorid-Elektrolyse. Es wird jedoch an keiner Stelle dieses Dokumentes auf eine Aufkonzentrierung der Alkalichlorid-haltigen Lösung hingewiesen.

Aufkonzentrierungsverfahren hingegen, wodurch dem Alkalichlorid-haltigen Abwasser Wasser entzogen wird, sind bekannt.

Gemäß der WO 01/38419 kann die Natriumchlorid-haltige Lösung mittels thermischer Verfahren eingedampft werden, so dass eine hoch konzentrierte Natriumchlorid-Lösung der Elektrolysezelle zugeführt werden kann. Die Eindampfung ist jedoch energieintensiv und kostspielig.

Auch kann beispielsweise die Umkehrosmose oder besonders bevorzugt die Membrandestillation oder Membrankontaktoren eingesetzt werden (siehe MELIN; RAUTENBACH, Membranverfahren; SPRINGER, BERLIN, 2003). Nachteilig hierbei ist der hohe Energiebedarf zur Überwindung der hohen osmotischen Drücke, wodurch die Wirtschaftlichkeit nichtmehr gegeben ist.

Die voranstehenden integrierten Verfahren haben allesamt den Nachteil, dass in Kombination mit einer Diarylcarbonat-Herstellung nur begrenzt konzentrierte NaCl-Lösungen (10 - 20 Gew%) der Elektrolyse zugeführt werden, so, dass eine Wiederverwertung von NaCl nur im beschränkten Umfang möglich ist oder die Aufkonzentrierung energieintensiv und kostspielig ist.

Ausgehend vom oben geschilderten Stand der Technik ist die Aufgabe der Erfindung ein Diarylcarbonat-Herstellungsverfahren bereitzustellen, welches Produkte in hoher Reinheit und guter Ausbeute liefert und eine Reduzierung der Umweltbelastung bzw. Abwasserproblematik in den Klärwerken durch maximierte Rückführung von Prozessabwasser-Lösungen, die aus der Diarylcarbonat-Produktion stammen, erreicht werden.

Weiterhin sollte bei dem Recycling die Umsetzung von Natriumchlorid zu Chlor und Natronlauge und gegebenenfalls Wasserstoff durch Elektrolyse mit minimalem Energieeinsatz und daher Resourcen-schonend erfolgen.

Die Aufgabe wird dadurch gelöst, dass im Verfahren die Verwertung von Natriumchlorid-haltigen Abwasserphasen durch eine vorgelagerte Aufkonzentrierung der NaCl-Lösung aus der Diarylcarbonat-Herstellung für die Elektrolyse mit Hilfe einer osmotischer Membrandestillation erfolgt.

Es wurde gefunden, dass die bei der kontinuierlichen Herstellung von Diarylcarbonaten durch Reaktion von Monophenolen und Phosgen in einem inerten Lösungsmittel in Gegenwart von Alkali und einem Stickstoffkatalysator in der Phasengrenzfläche anfallende Natriumchlorid-haltige Abwasserlösungen ohne aufwendige Reinigung nach pH-Einstellung auf einen Wert kleiner oder gleich 8 und einfacher Behandlung mit Aktivkohle direkt in einer osmotischen Membrandestillation aufkonzentriert werden können und einer elektrochemischen Oxidation des enthaltenen Natriumchlorids zu Chlor, Natronlauge und gegebenenfalls Wasserstoff zugeführt werden können, wobei das Chlor, zumindest teilweise, zur Herstellung des Phosgens rückgeführt werden kann.

Gegenstand der Erfindung ist daher ein Verfahren zur Herstellung von Diarylcarbonat und Verwertung der Abwasserphasen. Insbesondere besteht das Verfahren in der Aufkonzentrierung der Natriumchlorid-haltigen Abwasserphasen des Diphenylcarbonat-Herstellungsverfahren (DPC-Verfahren) zur Verarbeitung in einer nachgeschalteten Alkalichlorid-Elektrolyse durch osmotische Membran Destillation. Dabei kann gleichzeitig eine in der Elektrolyse gebildete Natronlauge verdünnt werden, die im Anschluss direkt als Edukt in das DPC-Verfahren eingesetzt werden kann.

Verfahren zur Herstellung von Diarylcarbonat umfassend folgenden Schritte:
a) Herstellung von Phosgen durch Umsetzung von Chlor mit Kohlenmonoxid,
b) Umsetzung des gemäß Schritt a) gebildeten Phosgens mit wenigstens einem Monophenol in Gegenwart einer wässrigen Alkali-haltigen Base, insbesondere einer Natrium-haltigen Base, gegebenenfalls eines Stickstoffkatalysators und gegebenenfalls eines organischen Lösungsmittels zu einem Diarylcarbonat und einer Alkalichlorid-haltigen, insbesondere einer Natriumchlorid-haltigen Reaktionsabwasserlösung,
c) Abtrennung und Aufarbeitung des in Schritt b) gebildeten Diarylcarbonats,
d) gegebenenfalls Abtrennung der Lösungsmittelreste und gegebenenfalls Katalysatorreste von der gemäß Schritt c) verbliebenen Alkalichlorid-haltigen Lösung, insbesondere durch Extraktion oder Strippen der Lösung mit Wasserdampf, und/oder Behandlung mit Adsorbentien, insbesondere mit Aktivkohle,
e) Osmotische Membrandestillation wenigstens eines Teils der gemäß Schritt c) oder d) verbliebenen Alkalichlorid-haltigen Lösung,
f) Elektrochemische Oxidation wenigstens eines Teils der Alkalichlorid-haltigen Lösung aus e) unter Bildung von Chlor, Alkalilauge und gegebenenfalls Wasserstoff.

In einer besonders bevorzugten Ausführungsform ist das Verfahren dadurch gekennzeichnet, dass beim Schritt c) oder d) vor der Behandlung mit Adsorbentien die Lösung auf einen pH-Wert kleiner oder gleich 8, bevorzugt 6 bis 8 eingestellt wird.

In einer weiteren besonderen Ausführungsform wird wenigstens ein Teil des gemäß Schritt f) hergestellten Chlors in die Herstellung von Phosgen gemäß Schritt a) zurückgeführt.

In einer weiteren bevorzugten Ausführungsform wird wenigstens ein Teil des gemäß Schritt f) hergestellten Alkalilauge in die osmotische Membrandestillation gemäß Schritt e) als Wasserakzeptor eingesetzt.

In einer weiteren besonders bevorzugten Ausführungsform wird wenigstens ein Teil der erhaltenen verdünnten Alkalilauge in die Herstellung von Diarylcarbonat gemäß Schritt b) zurückgeführt.

Die nach Abtrennung gemäß Schritt c) erhaltene Alkalichlorid-haltige Lösung (Reaktionsabwasser) kann einzeln oder vereinigt mit den Waschphasen aus der Aufarbeitung (Gesamtprozessabwasser), bevorzugt einzeln, nach Abtrennung von Lösungsmittelresten und gegebenenfalls Katalysatorresten gemäß Schritt d), in die osmotische Membrandestillation eingesetzt werden.

Besonders geeignete Monophenole zum Einsatz in dem neuen Verfahren sind Phenole der Formel (I) 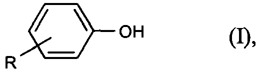 worin
- R: Wasserstoff, Halogen oder ein verzweigter oder unverzweigter C₁- bis C₉-Alkylrest oder Alkoxycarbonylrest ist.

Bevorzugt sind Phenol, Alkylphenole wie Kresole, p-tert.-Butylphenol, p-Cumylphenol, p-n-Octylphenol, p-iso-Octylphenol, p-n-Nonylphenol und p-iso-Nonylphenol, Halogenphenole wie p-Chlorphenol, 2,4-Dichlorphenol, p-Bromphenol und 2,4,6-Tribromphenol oder Salicylsäuremethylester. Besonders bevorzugt ist Phenol.

Das eingesetzte Alkali zur Bildung des Phenolats in Schritt b) kann beispielsweise eine Alkalilauge mit Hydroxiden aus der Reihe: Na-, K-, Li-Hydroxid sein, bevorzugt ist Natronlauge, und wird im neuen Verfahren vorzugsweise als 10 bis 55 Gew.%-ige Lösung eingesetzt. Die Alkalilauge wird besonders bevorzugt im Bereich von 1,0 bis 1,1 Moläquivalent bezogen auf Phenol, eingesetzt

Die Reaktion in Schritt b) kann durch Stickstoffkatalysatoren, wie tertiäre Amine, N-Alkylpiperidine oder Oniumsalze beschleunigt werden.

Der eingesetzte Amin-Katalysator kann offenkettig oder cyclisch sein, bevorzugt sind Tributhylamin, Triethylamin und N-Ethylpiperidin. Der Katalysator wird im erfindungsgemäßen Verfahren vorzugsweise als 1 bis 55 Gew.%-ige Lösung eingesetzt.

Die Konzentration des Katalysators beträgt bevorzugt 0,0001 mol bis 0,1 mol, bezogen auf das eingesetzte Monophenol.

Unter Oniumsalzen werden hier Verbindungen wie NR₄X verstanden, wobei R ein Alkyl und/oder Arylrest und/oder ein H sein kann und X ein Anion ist.

Phosgen kann in dem Verfahrensschritt b) flüssig, gasförmig oder gelöst in einem inerten Lösungsmittel eingesetzt werden.

In dem neuen Verfahren in Schritt b) bevorzugt verwendbare inerte organische Lösungsmittel sind beispielsweise Dichlormethan, Toluol, die verschiedenen Dichlorethane und Chlorpropanverbindungen, Chlorbenzol und Chlortoluol. Vorzugsweise wird Dichlormethan eingesetzt.

Die Reaktionsführung für Schritt b) erfolgt bevorzugt kontinuierlich und besonders bevorzugt in einer Pfropfenströmung ohne große Rückvermischung. Dies kann somit beispielsweise in Rohrreaktoren geschehen. Die Durchmischung der zwei Phasen (wässrige und organische Phase) kann durch eingebaute Rohrblenden, statische Mischer und/oder beispielsweise Pumpen realisiert werden. Die Reaktion gemäß Schritt b) kann einstufig oder zweistufig erfolgen, besonders bevorzugt zweistufig.

Verläuft das Verfahren in Schritt b) zweistufig, so wird bevorzugt in der ersten Stufe des erfindungsgemäßen Verfahrens die Reaktion durch Zusammenbringen der Edukte Phosgen, des inerten Lösungsmittels, welches bevorzugt erst als Lösungsmittel für das Phosgen dient, und des Monophenols, welches vorzugsweise zuvor bereits in der Alkalilauge gelöst ist, gestartet. Die Verweilzeit liegt typischerweise in der ersten Stufe im Bereich von 2 Sekunden bis 300 Sekunden, besonders bevorzugt im Bereich von 4 Sekunden bis 200 Sekunden. Der pH-Wert der ersten Stufe wird durch das Verhältnis von Alkalilauge/ Monophenol /Phosgen bevorzugt so eingestellt, dass der pH-Wert im Bereich von 11,0 bis 12,0, bevorzugt 11,2 bis 11,8, besonders bevorzugt bei 11,4 bis 11,6 liegt. Die Reaktionstemperatur der ersten Stufe wird durch Kühlung bevorzugt <40°C, besonders bevorzugt <35°C gehalten.

In einer zweiten Stufe des erfindungsgemäßen Verfahrens wird dann bevorzugt die Reaktion zum Diarylcarbonat komplettiert. Die Verweilzeit beträgt beim bevorzugten Prozess 1 Minute bis 2 Stunden, bevorzugt 2 Minuten bis 1 Stunde, ganz besonders bevorzugt 3 Minuten bis 30 Minuten. In der zweiten Stufe des bevorzugten Verfahrens wird durch permanente Kontrolle des pH-Werts (wird im kontinuierlichen Verfahren bevorzugt Online nach grundsätzlich bekannten Verfahren gemessen) und entsprechende Einstellung des pH-Wertes durch Zugabe der Alkalilauge geregelt. Die Menge zugeführten Alkalilauge wird insbesondere so eingestellt, dass der pH-Wert der Reaktionsmischung in der zweiten Verfahrensstufe im Bereich von 7,5 bis 10,5, bevorzugt 8 bis 9,5, ganz besonders bevorzugt 8,2 bis 9,3 liegt. Die Reaktionstemperatur der zweiten Stufe wird durch Kühlung bei bevorzugt <50°C, besonders bevorzugt <40°C, ganz besonders bevorzugt <35°C gehalten. Die in dieser Anmeldung aufgeführten allgemeinen oder in Vorzugsbereichen aufgeführten Parameter bzw. Erläuterungen können jedoch auch untereinander, also zwischen den jeweiligen Bereichen und Vorzugsbereichen beliebig kombiniert werden.

Im bevorzugten Verfahren wird in Schritt b) Phosgen in Bezug auf das Monophenol im MolVerhältnis von 1 : 2 bis 1 : 2,2 eingesetzt. Das Lösungsmittel wird so zugemischt, dass das Diarylcarbonat in einer 5 bis 60 %-igen Lösung, bevorzugt 20 bis 45 %-igen Lösung nach der Reaktion vorliegt.

Nach der Reaktion b) wird in Schritt c) bevorzugt die organische, den Diarylcarbonat enthaltende Phase üblicherweise mit einer wässrigen Flüssigkeit gewaschen und nach jedem Waschvorgang von der wässrigen Phase soweit wie möglich getrennt. Als Waschflüssigkeit werden wässrige Flüssigkeiten zur Abtrennung des Katalysators, z.B. eine verdünnte Mineralsäure wie HCl oder H₃PO₄, bevorzugt HCl, und zur weiteren Reinigung vollentsalztes Wasser eingesetzt. Die Diarylcarbonatlösung ist nach der Wäsche und Abtrennung der Waschflüssigkeit üblicherweise trüb. Die Konzentration von HCl oder H₃PO₄ in der Waschflüssigkeit kann beispielsweise 0,5 bis 1,0 Gew.-% betragen. Die organische Phase wird beispielhaft und vorzugsweise zweimal gewaschen.

Als Phasentrennvorrichtungen zur Abtrennung der Waschflüssigkeit von der organischen Phase können grundsätzlich dem Fachmann bekannte Trenngefäße, Phasenseparatoren, Zentrifugen oder Coalescer oder auch Kombinationen dieser Einrichtungen verwendet werden.

Es können mit dem erfindungsgemäßen Verfahren ohne Berücksichtigung des noch abzutrennenden Lösungsmittels hohe Reinheitsgrade des Diarylcarbonats von >99,85 % erhalten werden.

In einer bevorzugten Ausführungsform wird das Diarylcarbonat nach der Synthese des Diarylcarbonats in Form seiner Lösung in dem bei der Synthese verwendeten organischen Lösungsmittel, beispielsweise Methylenchlorid, abgetrennt.

Zum Erhalt des hochreinen Diarylcarbonats wird das Lösungsmittel anschließend verdampft. Das Verdampfen kann in mehreren Verdampferstufen erfolgen. Beispielsweise erfolgt dies durch eine oder mehrere hintereinander geschaltete Destillationskolonnen bei der das Lösungsmittel vom Diarylcarbonat abgetrennt wird.

Der Reinigungsschritt c) kann einstufig oder mehrstufig erfolgen. Die Stufen können beispielsweise kontinuierlich so geführt werden, dass die Sumpftemperatur bei der Destillation 150°C bis 310°C, bevorzugt 160 bis 230°C beträgt. Der zur Durchführung dieser Destillation angewendete Druck beträgt dabei insbesondere 0,1 kPa bis 100 kPa (1 bis 1000 mbar), bevorzugt 0,5 kPa bis 10 (5 bis 100 mbar) kPa.

Die so gereinigten Diarylcarbonate zeichnen sich durch besonders hohe Reinheit (GC >99,95%) und extrem gutes Umesterungsverhalten aus, so dass hieraus anschließend ein Polykarbonat in exzellenter Qualität hergestellt werden kann.

Die Verwendung der Diarylcarbonate zur Herstellung von aromatischen Oligo-/Polycarbonaten nach dem Schmelzumesterungsverfahren ist literaturbekannt und beispielsweise in der Encyclopedia of Polymer Science, Vol. 10 (1969), Chemistry and Physics of Polycarbonates, Polymer Reviews, H. Schnell, Vol. 9, John Wiley and Sons, Inc. (1964) oder der US-A 5 340 905 beschrieben.

Die verbleibende wässrige Lösung gemäß Schritt c) wird bevorzugt von leicht flüchtigen organischen Verunreinigungen, wie z.B. Reste des bei der Synthese verwendeten organischen Lösungsmittels und gegebenenfalls verbleibendem Katalysator beispielsweise durch Destillation oder Wasserdampfstrippen befreit. Es verbleibt dann ein Abwasser mit einem Gehalt an gelöstem Natriumchlorid (10-20 Gew.%) und gelösten Natriumcarbonaten (0,3-1,5 Gew.-%). Die Carbonate entstehen dabei z.B. durch Hydrolyse des Phosgens als Nebenreaktion der Diarylcarbonat-herstellung. Das Abwasser ist weiterhin mit organischen Verbindungen belastet, z.B. mit Phenolen (z.B. unsubstituiertes Phenol, Alkylphenole).

In einer besonders bevorzugten Ausführungsform erfolgt eine Behandlung des vorgereinigten Abwassers mit Adsorbentien, bevorzugt mit Aktivkohle.

Gemäß einem bevorzugten Verfahren wird die Erniedrigung des pH-Wertes im Prozessschritt c) oder d) mit Salzsäure oder Chlorwasserstoff durchgeführt.

Eine bevorzugte Verfahrensvariante ist, dass dem Alkalichlorid-haltigen Abwasser durch ein Aufkonzentrierungsverfahren Wasser entzogen wird.

Die Aufkonzentrierung von NaCl-Lösungen durch osmotische Destillation ist energieschonend, insbesondere dann, wenn die aus der NaCl-Elektrolyse stammende NaOH-Lösung als Wasserakzeptor eingesetzt wird. Dies hat besonders dann Vorteile, wenn in der DPC-Produktion eine verdünnte Natronlauge eingesetzt wird, wobei dann zusätzlich das Wasser zur Verdünnung der Natronlauge eingespart werden kann.

Besonders bevorzugt ist daher ein Verfahren, dadurch gekennzeichnet, dass die Alkalichlorid-haltige Lösung aus d) vor der Elektrolyse f) mittels osmotische Destillation mit Natronlauge als Wasserakzeptor aufkonzentriert wird (Schritt e).

Durch Kombination von erfindungsgemäßem Betrieb der Elektrolysezellen und Aufkonzentrierungsverfahren können theoretisch bis zu 100 % des Natriumchlorids aus dem Abwasser wieder gewonnen werden.

Die osmotische Destillation erfolgt durch molekulare und gegebenenfalls Knudsen Diffusion von Wasser-dampf durch eine Membran. Dadurch ist die Diffusionsrate abhängig vom Unterschied zwischen den Wasserdampfdrücken auf beiden Seiten der Membran, sowie deren Porosität, Stärke und Gewundenheit.

Um eine effiziente Aufkonzentrierung zu ermöglichen, sollte als Wasser-Akzeptor eine konzentrierte Lösung eines Alkalihydroxids, bevorzugt Natrium- oder Kaliumhydroxid, besonders bevorzugt Natriumhydroxid verwendet werden.

Für das erfindungemäße Verfahren sollte als Membranwerkstoff ein chemisch beständiger Werkstoff wie z.B. Polypropylen eingesetzt werden. Die Membranen sollten bevorzugt Kapillarmembranen mit einer Kapillarlänge von 30-6000µm und einem Durchmesser von 0,01 bis 0,7µm eingesetzt werden.

Besonders geeignete Membranen sind lipophile Membrane, wie beispielsweise Accurel PP 50/200, Accurel PP 50/280, Accurel PP 150/330, Accurel PP Q3/2 oder Accurel S 6/2 der Firma Membrana.

Das Verfahren wird bevorzugt so betrieben, dass die osmotische Destillation bei einer Temperatur von 10 bis 100°C, vorzugsweise von 20 bis 50°C betrieben wird. Die Temperatur der eingesetzten Natronlauge kann dabei höher sein als die des NaCl-haligen Abwassers.

Die osmotische Destillation wird bei einem Absolutdruck von 100 kPa bis 140 kPa (1, bis 1,4 bar), bevorzugt bei einem Druck von 110 kPa bis 120 kPa (1,1 bis 1,2 bar) betrieben.

Die Druckverhältnisse zwischen Alkalichloridlösung und Alkalihydroxidlösung werden insbesondere so gewählt, dass der osmotische Druck der Alkalichloridlösung höher ist als der Druck der Alkalihydroxidlösung.

Der Differenzdruck zwischen Alkalichloridlösung und Alkalihydroxidlösung sollte in einem besonders bevorzugten Verfahren 2 MPa bis 15 MPa (20 bis 150 bar), vorzugsweise 3 MPa bis 10 MPa (30 bis 100 bar) betragen.

Das Verfahren der Alkalichlorid-Elektrolyse wird im Folgenden näher beschrieben. Die nachstehende Beschreibung ist in Bezug auf die Elektrolyse von Natriumchlorid beispielhaft anzusehen, da im Verfahren wie bereits oben ausgeführt wurde grundsätzlich jedes Alkalichlorid zum Einsatz kommen kann (insbesondere Li-, Na-, KCl), die Verwendung von Natriumchlorid bzw. Natronlauge in den vorgehenden Stufen aber die bevorzugte Ausführung des Verfahrens ist.

Üblicherweise werden z.B. zur Elektrolyse von Natriumchlorid-haltigen Lösungen Membranelektrolyseverfahren eingesetzt (siehe hierzu Peter Schmittinger, CHLORINE, Wiley-VCH Verlag, 2000). Hierbei wird eine zweigeteilte Elektrolysezelle eingesetzt, die aus einem Anodenraum mit einer Anode und einem Kathodenraum mit einer Kathode besteht. Anoden- und Kathodenraum werden von einer Ionenaustauschermembran getrennt. In den Anodenraum wird eine Natriumchlorid-haltige Lösung mit einer Natriumchlorid-Konzentration von üblicherweise mehr als 300 g/l eingeführt. An der Anode wird das Chlorid-Ion zu Chlor oxidiert, dass mit der abgereicherten Natriumchlorid-haltigen Lösung (ca. 200 g/l) aus der Zelle geführt wird. Die Natrium-Ionen wandern unter Einfluss des elektrischen Feldes durch die Ionenaus-tauschermembran in den Kathodenraum. Bei dieser Wanderung nimmt jedes mol Natrium je nach Membran von 3,5 bis 4,5 mol Wasser mit. Dies führt dazu, dass der Anolyt an Wasser verarmt. Im Gegensatz zum Anolyten wird auf der Kathodenseite durch die Elektrolyse von Wasser zu Hydroxid-Ionen und Wasserstoff Wasser verbraucht. Das mit den Natrium-Ionen in den Katholyten gelangende Wasser reicht aus, um die Natronlauge-Konzentration im Auslauf auf 31-32 Gew.-% zu halten, dies bei einer Einlaufkonzentration von 30% und einer Stromdichte von 4 kA/m². Im Kathodenraum wird Wasser elektrochemisch reduziert, wobei Hydroxid-Ionen und Wasserstoff entstehen.

Alternativ kann als Kathode auch eine Gasdiffusionselektrode eingesetzt werden, an der Sauerstoff mit Elektronen zu Hydroxid-Ionen umgesetzt wird, wobei kein Wasserstoff entsteht. Mit den über die Ionenaustauschermembran in den Kathodenraum gelangten Natrium-Ionen bilden die Hydroxid-Ionen Natronlauge. In die Kathodenkammer wird üblicherweise eine Natronlauge mit einer Konzentration von 30 Gew.-% zugeführt und eine Natronlauge mit einer Konzentration von 31-32 Gew.-% abgeführt. Ziel ist es eine möglichst hohe Konzentration an Natronlauge zu erreichen, da üblicherweise die Natronlauge als 50 Gew.-%ige Lauge gelagert oder transportiert wird. Handelsübliche Membranen sind jedoch derzeit nicht beständig gegen eine Lauge mit einer höheren Konzentration als 32 Gew.-%, so dass die Natronlauge durch thermische Eindampfung aufkonzentriert werden muss.

Im Falle der Natriumchlorid-Elektrolyse wird über diese Natriumchlorid-haltige Lösung zusätzliches Wasser in den Anolyten eingebracht, jedoch nur Wasser über die Membran in den Katholyten ausgetragen. Wird mehr Wasser über die Natriumchlorid-haltige Lösung eingebracht als zum Katholyten transportiert werden kann, verarmt der Anolyt an Natriumchlorid und die Elektrolyse kann nicht kontinuierlich betrieben werden. Bei sehr geringen Natriumchlorid-Konzentrationen würde die Nebenreaktion der Sauerstoffbildung einsetzen.

Um maximale Mengen an Natriumchlorid-haltigen Lösungen wirtschaftlich der Natriumchlorid-Elektrolyse zuzuführen, kann es nützlich sein, dass der Wassertransport über die Membran erhöht wird. Dieser kann durch Auswahl geeigneter Membranen erfolgen, wie in der US-A-4025405 beschrieben. Der Effekt eines erhöhten Wassertransportes ist, dass auf die sonst übliche Wasserzugabe zur Aufrechterhaltung der Laugekonzentration verzichtet werden kann.

Gemäß der US-A-3 773 634 kann bei hohem Wassertransport durch die Membran die Elektrolyse dann betrieben werden, wenn eine Laugekonzentration von 31 bis 43 Gew.-% und eine Natriumchlorid-Konzentration von 120 bis 250 g/l eingesetzt wird.

Gemäß dem bevorzugten Verfahren erfolgt die Abtrennung d) des Natriumchlorid-haltigen Reaktionsabwassers nach der Phasentrennung und der Entfernung des Lösungsmittels und gegebenenfalls verwendeten Katalysators, durch Extraktion oder Strippung mit Wasserdampf und, nach der pH-Einstellung, durch eine Aktivkohlebehandlung.

Hiernach kann das Alkalichlorid-haltige Abwasser direkt der osmotische Destillation e) zugeführt werden.

Gegenüber dem Stand der Technik (WO 03/70639), in dem max. 26 % des im Abwasser der DPC-Produktion vorhandenen Natriumchlorids bei der NaCl-Elektrolyse eingesetzt werden kann, können durch das erfindungsgemäße Verfahren mehr als 26 % des Natriumchlorids aus dem Abwasser zurück gewonnen werden.

Das neue Verfahren kann auch mit einer Alkalichlorid-Elektrolyse durchgeführt werden, bei der kein Wasserstoff an der Kathode erzeugt wird, sondern die Kathode durch eine Gasdiffusionselektrode ersetzt wird, an der Sauerstoff zu Hydroxid-Ionen reduziert wird.

Wenn z.B. in einem Verbundstandort kein Wasserstoff für chemische Reaktionen benötigt wird, kann auf das Zwangsanfallprodukt Wasserstoff verzichtet werden. Vorteil ist eine Energieeinsparung bei der Elektrolyse, die durch die niedrigere Elektrolysespannung bei Einsatz einer Gasdiffusionselektrode zurückzuführen ist.

Die aus der DPC-Produktion gelangende Natriumchlorid-haltige Lösung weist üblicherweise einen Natriumchlorid-Gehalt von bis zu 18 Gew.-% auf, soweit es sich um das Reaktionsabwasser handelt. Wird das Reaktionsabwasser mit dem Waschwasser vereinigt so beträgt die NaCl-Konzentration beispielsweise ca. 13 Gew.-%. Liefert die Elektrolyse das Chlor und die Natronlauge ausschließlich für die DPC-Produktion, so kann das Natriumchlorid-haltige Abwasser nur zu einem geringen Teil in der Elektrolyse eingesetzt werden. So können bei den üblichen Ionenaustauschermembranen und den Standardbetriebsparametern der Natriumchlorid-Elektrolyse nur max. 26 % des Natriumchlorids eines 17 Gew.-%igen Natriumchlorid-haltigen DPC-Abwassers eingesetzt werden. Die Standardbetriebsparameter der NaCl-Elektrolyse sind eine Solekonzen-tration im Ablauf von 200 bis 240g/l und eine NaOH-Konzentration von 31-32 Gew.-%. Ein vollständiges Recycling des anfallenden Natriumchlorids ist daher bisher nicht möglich. Eine Aufkonzentrierung durch thermische Verdampfung des Wassers ist derzeit nicht wirtschaftlich, da das Natriumchlorid als sehr preiswertes Produkt zur Verfügung steht.

Mit dem erfindungsgemäßen Verfahren können deutlich mehr als 26 % des Natriumchlorid aus anfallenden Abwässern, bei einer Konzentration von 17 Gew.-% recycelt werden, sofern die Natriumchlorid-Elektrolyse ausschließlich das Chlor und die Natronlauge für die DPC-Produktion bereitstellt. Üblicherweise werden Natriumchlorid-Elektrolysen an chemischen Verbundstandorten mit mehreren Chlorverbrauchern betrieben, so dass nicht von allen Verbrauchern eine Natriumchlorid-haltige Lösung zum Recycling bereit steht. Der Anteil an wieder verwertbarem Natriumchlorid aus dem Abwasser steigt, wenn die Natriumchlorid-Elektrolyse die Natronlauge und das Chlor nicht ausschließlich für die Diarylcarbonat-Produktion bereitstellen muss.

Eine weitere bevorzugte Variante des neuen Verfahrens ist, dass das Abwasser der Diarylcarbonatherstellung durch festes Alkalichlorid aufkonzentriert und der Alkalichlorid-Elektrolyse zugeführt wird. Hierdurch könnten mehr als 50% des Alkalichlorids aus dem DPC-Abwasser wieder verwendet werden.

Dies setzt jedoch voraus, dass das Chlor und die Alkalilauge nicht ausschließlich für die Diarylcarbonat-Produktion eingesetzt werden.

Besonders bevorzugt wird ein Alkalichlorid-haltiges Abwasser bei der Elektrolyse f) eingesetzt bzw. zugeführt dessen pH-Wert kleiner als 7 beträgt. Die pH-Wert Einstellung erfolgt vorzugsweise mit Salzsäure, kann aber auch mit gasförmigem Chlorwasserstoff erfolgen.

Gemäß einem weiteren bevorzugten Verfahren wird die NaCl-Elektrolyse so betrieben, dass die NaCl-Lösung, die aus der Zelle gelangt, eine NaCl-Konzentration von weniger als 200 g/l aufweist. Parallel hierzu kann die aus der Zelle ablaufende Laugenkonzentration weniger als 30 Gew.-% betragen.

Der Wassertransport über die Ionenaustauschermembran hängt nicht nur von den Betriebsparametern ab, sondern auch von dem eingesetzten Membran-Typ. Gemäß dem erfindungsgemäßen Verfahren werden bevorzugt solche Ionenaustauschermembranen eingesetzt, die unter den erfindungsgemäßen Bedingungen der Natriumchlorid- und Laugekonzentration einen Wassertransport durch die Membran von mehr als 4,5 mol Wasser je mol Natrium ermöglichen.

Die Stromdichte wird dabei auf die Membranfläche berechnet und beträgt insbesondere 2 bis 6 kA/m². Besonders bevorzugt werden Anoden mit größerer Oberfläche eingesetzt. Unter Anoden mit größerer Oberfläche sind solche zu verstehen, bei denen die physikalische Oberfläche deutlich höher ist als die projekzierte Oberfläche. Anoden mit größerer Oberfläche sind z.B. Schaum- oder Filz-artig aufgebaute Elektroden. Hierdurch wird anodische eine sehr große Elektrodenoberfläche angeboten und die lokale Stromdichte stark erniedrigt. Die Oberfläche der Anode ist bevorzugt so zu wählen, das die lokale Stromdichte bezogen auf die physikalische Oberfläche der Elektrode kleiner als 3 kA/m² beträgt. Je größer die Oberfläche und je niedriger die lokale Stromdichte, desto gering kann die Natriumchlorid-Konzentration in der Sole gewählt werden und desto höher ist der Anteil an Natriumchlorid aus dem Abwasser, der recycelt werden kann.

Der pH-Wert des Alkalichlorid-haltigen Abwassers sollte vor der Elektrolyse f) bevorzugt kleiner als 7 betragen, besonders bevorzugt von 0,5 bis 6.

Die Alkalichlorid-Elektrolyse sollte so betrieben werden, dass die Alkalichlorid-Konzentration der aus der Zelle gelangenden Alkalichlorid-Lösung von 100 bis 280 g/l Natriumchlorid und/oder dass die Konzentration der Alkalilauge, die aus der Zelle gelangt 13 bis 33 Gew.-% beträgt.

Besonders bevorzugt sind Konzentrationen, die den Betrieb der Zelle bei niedrigeren Spannungen ermöglichen. Hierzu sollte die Konzentration der aus der Zelle gelangenden Alkalichlorid-Lösung vorzugsweise von 110 bis 220 g/l Alkalichlorid und/oder die Konzentration der Alkalilauge, die aus der Zelle gelangt 20 bis 30 Gew.-% betragen.

Die eingesetzten Ionenaustauschermembranen bei der Elektrolyse sollten bevorzugt einen Wassertransport je mol Natrium von mehr als 4,0 mol H₂O/mol Natrium aufweisen, besonders bevorzugt 4,5 bis 6,5 mol H₂O/mol Natrium.

Das Verfahren wird bevorzugt so betrieben, dass die Elektrolyse f) bei einer Temperatur von 70 bis 100°C, vorzugsweise bei 80 bis 95°C betrieben wird.

Die Elektrolyse wird bei einem Absolutdruck von 100 kPa bis 140 kPa (1 bis 1,4 bar), bevorzugt bei einem Druck von 110 kPa bis 120 kPa (1,1 bis 1,2 bar) betrieben.

Die Druckverhältnisse zwischen Anoden- und Kathodenraum werden insbesondere so gewählt, dass der Druck im Kathodenraum höher ist als der Druck im Anodenraum.
Der Differenzdruck zwischen Kathoden- und Anodenraum sollte in einem besonders bevorzugten Verfahren 2 kPa bis 15 kPa (20 bis 150 mbar), vorzugsweise 3 kPa bis 10 kPa (30 bis 100 mbar) betragen.

Bei niedrigeren Alkalichlorid-Konzentrationen können auch spezielle Anodencoatings eingesetzt werden. Insbesondere kann das Coating der Anode neben Rutheniumoxid auch weitere Edelmetallkomponenten der 7. und 8. Nebengruppe des Periodensystems der Elemente enthalten. Beispielsweise kann das Anodencoating mit Palladiumverbindungen dotiert werden. Ebenso sind Coatings auf Basis von Diamanten einsetzbar.

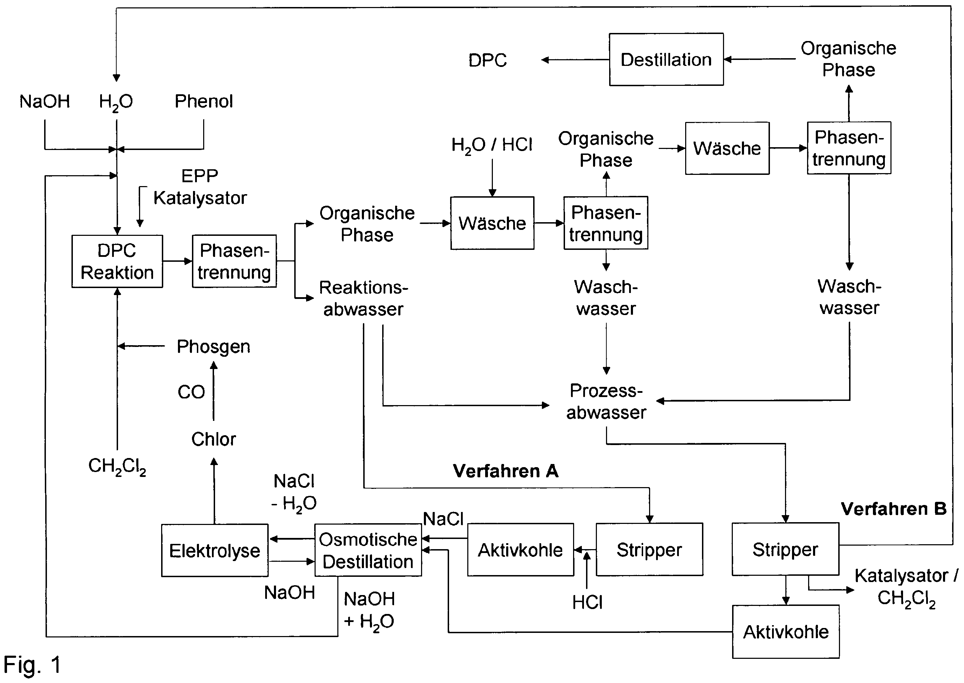

Fig. 1: Schematische Darstellung eines erfindungsgemäßen Verfahrens zur Herstellung von DPC durch Phasengrenzflächenphosgenierung von Na-Phenolat und Verwertung der Natriumchlorid-haltigen Abwasserphasen für die Elektrolyse durch Aufkonzentrierung mit osmotischer Membrandestillation unter gleichzeitiger Verdünnung der aus der Elektrolyse erhaltenen Natronlauge für das Diphenylcarbonat-Herstellungsverfahren.

Die nachfolgenden Beispiele sollen die vorliegende Erfindung illustrieren ohne sie jedoch einzuschränken.

### Beispiele

Die Beispiele sollen das erfindungsgemäße Verfahren anhand der Aufkonzentrierung der bei der Herstellung von Diphenylcarbonat anfallenden Natriumchlorid-haltigen Abwasserphasen und gleichzeitige Verdünnung der aus der Elektrolyse erhaltenen Natronlauge für das Diphenylcarbonat-Herstellungsverfahren (DPC-Verfahren) durch osmotische Membrandestillation und Elektrolyse der erhaltenen Natriumchlorid-haltigen Lösungen darstellen.

### Beispiel 1

### a) Herstellung und Aufarbeitung von Diphenylcarbonat

In einen senkrecht stehenden, gekühlten Rohrreaktor wurde kontinuierlich ein Gemisch aus 145,2 kg/h 14,5 %-iger Natronlauge, hergestellt durch Verdünnung von 65,8 kg/h einer 32,0 %-igen Natronlauge mit 79,4 kg/h vollentsalztem Wasser (VE-Wasser), und 48,3 kg/h Phenol mit einer Lösung von 86,2 kg/h Methylenchlorid und 27,5 kg/h Phosgen (8 Mol % Überschuss bzgl. auf Phenol) zusammengebracht. Dieses Reaktionsgemisch wurde auf eine Temperatur von 33°C gekühlt und nach einer mittleren Verweilzeit von 15 Sekunden ein pH-Wert von 11,5 gemessen. Zu diesem Reaktionsgemisch wurden nun in der zweiten Stufe des Verfahrens 5,4 kg/h 50,0 %-iger NaOH zudosiert, so dass der pH-Wert der zweiten Reaktionsstufe nach einer weiteren Verweilzeit von 5 Minuten 8,5 beträgt. In der zweiten Stufe des Verfahrens wurde das Reaktionsgemisch durch das Durchleiten eines mit Verengungen versehen Rohres ständig gemischt. Die Reaktionstemperatur wurde nach erneuter Zugabe der NaOH durch Kühlen auf 30°C eingestellt. Nach Abtrennung der organischen von der wässrigen Phase (Reaktionsabwasser) wurde die DPC-Lösung mit 0,6 %-iger Salzsäure und Wasser gewaschen. Nach Entfernung des Lösungsmittels wurde 99,9 %-iges Diphenylcarbonat erhalten. Das Reaktionsabwasser wurde nicht mit den Waschphasen vereinigt und wurde durch Strippen mit Wasserdampf von Lösungsmittelresten und Katalysator befreit. Nach Neutralisierung (pH 7) mit Salzsäure und Behandlung mit Aktivkohle enthielt das Reaktionsabwasser 17,0 % NaCl und < 2 ppm Phenol.

Das Abwasser konnte ohne weitere Reinigung der osmotischen Destillation zugeführt werden.

### b) Aufkonzentrierung der Natriumchlorid-Lösung durch osmotische Membrandestillation

Das Reaktionsabwasser aus a) wurde der osmotische Membrandestillation zugeführt.

Die osmotische Destillation wurde in einem Modul, welche eine Membranfläche von 1,1 m² aufwies, durchgeführt. Eingesetzt wurde eine Membran des Typs Accurel PP 150/330 der Firma Membrana. Bei Raumtemperatur wurden 1,7 l der 17,0 % Natriumchlorid-haltigen Reaktionsabwasserlösung aus a) in das Modul gepumpt, während gleichzeitig im Gleichstrom 0,6 L 32,0 %-ige Natronlauge als Wasseraufnehmende Phase eindosiert wurden. Die Konzentration der aus der Zelle austretende NaCl-haltigen Lösung betrug 21,9 Gew.-% NaCl, während die entnommene NaOH-Lösung eine Konzentration von 16,4 Gew.-% aufzeigte.

### c) Elektrochemische Oxidation der Natriumchlorid-Lösung aus der osmotischen Destillation

Die Elektrolyse wurde in einer Laborelektrolysezelle mit einer Anodenfläche von 0,01m² durchgeführt. Die Stromdichte betrug 4 kA/m², Temperatur Auslauf Kathodenseite 88°C, Temperatur Auslauf Anodenseite 89°C. Eingesetzt wurde eine Elektrolysezelle mit Standard Anoden- und Kathoden-coating der Fa. DENORA, Deutschland. Eingesetzt wurde eine Ionenaustauscher-membran der Fa. DuPont Nafion 982 WX. Die Elektrolysespannung betrug 3,02 V. Durch die Anodenkammer wurde eine Natriumchlorid-haltige Lösung mit einem Massenstrom von 0,8 kg/h umgepumpt. Die Konzentration der der Anodenkammer zugeführten Lösung betrug 25,0 Gew.-% NaCl. Aus der Anodenkammer konnte eine 18,8 Gew.%-ige NaCl-Lösung entnommen werden. Der aus der Anodenkammer entnommenen NaCl-Lösung wurden 0,152 kg/h an 21,9 Gew.%-igen Reaktionsabwasser aus der Diphenylcarbonatherstellung unter b) und 0,054 kg/h festes Natriumchlorid zugegeben. Die Lösung wurde anschließend wieder in die Anoden-kammer eingespeist. Der Wassertransport über die Membran betrug 3,8 mol Wasser je mol Natrium.

Auf der Kathodenseite wurde eine Natronlauge mit einem Massenstrom von 0,653 kg/h umgepumpt. Die Konzentration der in die Kathodenseite eingespeisten Natronlauge betrug 30,0 Gew.-% NaOH, die aus der Kathodenseite entnommene Natronlauge hatte eine Konzentration von 32,7 Gew.-% NaOH. 0,182 kg/h der 32,7 %-igen Lauge wurden dem Volumenstrom entnommen, der Rest wurde mit 0,0539 kg/h Wasser aufgestockt und wieder in das Kathodenelement zurückgeführt.

37,8 % des umgesetzten Natriumchlorids stammten aus dem Reaktionsabwasser der DPC-Herstellung.

### d) Rückführung der verdünnten Natriumhydroxid-lösung aus der osmotischen Destillation in die DPC-Herstellung

Die verdünnte Natriumhydroxid-Lösung aus b) wurde der Diphenylcarbonatherstellung zugeführt. Ein Gemisch aus 145,2 kg/h 14,5%-iger Natronlauge, hergestellt durch Verdünnung von 128,4 kg/h der 16,4 %-igen Natronlauge aus b) mit 16,8 kg/h vollentsalztem Wasser (VE-Wasser), und 48,3 kg/h Phenol wurde mit einer Lösung von 86,2 kg/h Methylenchlorid und 27,5 kg/h Phosgen (8 Mol-% Überschuss bzgl. auf Phenol) zusammengebracht und verfahren wie in 1a) beschrieben. Durch die Verwendung der 16,4 %-igen statt der üblichen 32,0 %-igen Natriumhydroxid-Lösung kann 62,7 kg/h (78,9 %) VE-Wasser eingespart werden.

### Beispiel 2

### a) Herstellung und Aufarbeitung von Diphenylcarbonat

Es wurde verfahren wie in Beispiel 1a beschrieben, nur wurde das Reaktionsabwasser mit den Waschphasen zu Gesamtprozessabwasser vereinigt und durch Strippen mit Wasserdampf von Lösungsmittelresten und Katalysator befreit. Nach Neutralisierung mit Salzsäure und Behandlung mit Aktivkohle enthielt das Gesamtprozessabwasser 13,0 % NaCl und < 2 ppm Phenol.

Das Abwasser konnte ohne weitere Reinigung der osmotischen Membrandestillation zugeführt werden.

### b) Aufkonzentrierung der Natriumchlorid-Lösung durch osmotische Membrandestillation

Die osmotische Destillation wurde in einem Modul mit einem 1,1 m² Membranmodul, ausgestattet mit einem Accurel PP 150/330 Membran der Firma Membrana, bei Raumtemperatur durchgeführt. Das 13,0 % Natriumchlorid-haltigen Prozessabwasser aus a) wurde mit einem Massenstrom von 21,0 l/h in die Laborzelle gepumpt, während 12,1 l/h 32,0 %-ige Natronlauge als Wasser-Akzeptor in Gleichstrom eindosiert wurde. Die Konzentration der aus der Zelle austretende Prozessabwasserlösung betrug 24,1 Gew.% NaCl, während die entnommene NaOH-Lösung auf 16,1 Gew.-% abgereichert war.

Die aus der Zelle austretende aufkonzentrierte Gesamtprozessabwasserlösung kann ohne weitere Reinigung der Elektrolyse zugeführt werden.

### c) Elektrochemische Oxidation der Natriumchlorid-Lösung aus der osmotischen Destillation

Die Elektrolyse wurde in einer Laborelektrolysezelle mit einer Anodenfläche von 0,01m² durchgeführt. Die Stromdichte betrug 4 kA/m², Temperatur Auslauf Kathodenseite 88°C, Temperatur Auslauf Anodenseite 89°C. Eingesetzt wurde eine Elektrolysezelle mit Standard Anoden- und Kathodencoating der Fa. DENORA, Deutschland. Eingesetzt wurde eine Ionenaus-tauschermembran der Fa. DuPont Nafion 982 WX. Die Elektrolysespannung betrug 3,02 V. Durch die Anodenkammer wurde eine Natriumchlorid-haltige Lösung mit einem Massenstrom von 0,8 kg/h umgepumpt. Die Konzentration der der Anodenkammer zugeführten Lösung betrug 25,0 Gew.-% NaCl. Aus der Anodenkammer konnte eine 18,8 Gew.-%ige NaCl-Lösung entnommen werden. Der aus der Anodenkammer entnommenen NaCl-Lösung wurden 0,157kg/h an 24,1 Gew.%-igem angereicherten Gesamtprozessabwasser aus der osmotische Destillation unter b) und 0,505 kg/h festes Natriumchlorid zugegeben. Die Lösung wurde anschließend wieder in die Anodenkammer eingespeist. Der Wassertransport über die Membran betrug 3,8 mol Wasser je mol Natrium.

Auf der Kathodenseite wurde eine Natronlauge mit einem Massenstrom von 0,653 kg/h umgepumpt. Die Konzentration der in die Kathodenseite eingespeisten Natronlauge betrug 30,0 Gew.% NaOH, die aus der Kathodenseite entnommene Natronlauge hatte eine Konzentration von 32,7 Gew.-% NaOH. 0,182 kg/h der 32,7%-igen Lauge wurden dem Volumenstrom entnommen, der Rest wurde mit 0,0539kg/h Wasser aufgestockt und wieder in das Kathodenelement zurückgeführt. 42,8 % des umgesetzten Natriumchlorids stammten aus dem DPC-Gesamtprozessabwasser.

### d) Rückführung der verdünnten Natriumhydroxid-lösung aus der osmotischen Destillation in die DPC-Herstellung

Die verdünnte Natriumhydroxid-Lösung aus b) wurde der Diphenylcarbonatherstellung zugeführt. Ein Gemisch aus 145,2 kg/h 14,5 %-iger Natronlauge, hergestellt durch Verdünnung von 130,8 kg/h der 16,1 %-igen Natronlauge aus b) mit 14,4 kg/h vollentsalztem Wasser (VE-Wasser), und 48,3 kg/h Phenol wurde mit einer Lösung von 86,2 kg/h Methylenchlorid und 27,5 kg/h Phosgen (8 Mol-% Überschuss bzgl. auf Phenol) zusammengebracht und verfahren wie in 1a) beschrieben.

Durch die Verwendung der 16,1 %-igen statt der üblichen 32,0 %-igen Natriumhydroxid-Lösung konnten 65,0 kg/h (81,8 %) VE-Wasser eingespart werden.

### Beispiel 3

### a) Herstellung und Aufarbeitung von Diphenylcarbonat

Es wurde verfahren wie in Beispiel 2a beschrieben, wobei das Reaktionsabwasser mit den Waschphasen zu Gesamtprozessabwasser vereinigt und durch Strippen mit Wasserdampf von Lösungsmittelresten und Katalysator befreit. Nach Neutralisierung mit Salzsäure und Behandlung mit Aktivkohle enthielt das Gesamtprozessabwasser 13,0 % NaCl und < 2 ppm Phenol.

Das Abwasser konnte ohne weitere Reinigung der osmotischen Membrandestillation zugeführt werden.

### b) Aufkonzentrierung der Natriumchlorid-Lösung durch osmotische Membrandestillation

Die osmotische Membrandestillation wurde kontinuierlich in einer Laborzelle mit einem 2 m² Membranmodul, ausgestattet mit einem Accurel PP 150/330 Membran der Firma Membrana, bei Raumtemperatur durchgeführt. Das 13,0 % Natriumchlorid-haltigen Prozessabwasser aus a) wurde mit einem Massenstrom von 3,0 l/h in die Laborzelle gepumpt, während 3,0 l/h 31,4 %-ige Natronlauge als Wasser-Akzeptor in Gleichstrom eindosiert wurde. Die Konzentration der aus der Zelle austretende Prozessabwasserlösung betrug 19,5 Gew.% NaCl, während die entnommene NaOH-Lösung auf 20,0 Gew.-% abgereichert war.

Die aus der Zelle austretende aufkonzentrierte Prozessabwasserlösung konnte ohne weitere Reinigung der Elektrolyse zugeführt werden.

### c) Elektrochemische Oxidation der Natriumchlorid-Lösung aus der osmotischen Destillation

Die Elektrolyse wurde in einer Laborelektrolysezelle mit einer Anodenfläche von 0,01m² durchgeführt. Die Stromdichte betrug 4 kA/m², Temperatur Auslauf Kathodenseite 88°C, Temperatur Auslauf Anodenseite 89°C. Eingesetzt wurde eine Elektrolysezelle mit Standard Anoden- und Kathodencoating der Fa. DENORA, Deutschland. Eingesetzt wurde eine Ionenaus-tauschermembran der Fa. DuPont Nafion 982 WX. Die Elektrolysespannung betrug 3,02 V. Durch die Anodenkammer wurde eine Natriumchlorid-haltige Lösung mit einem Massenstrom von 0,8 kg/h umgepumpt. Die Konzentration der der Anodenkammer zugeführten Lösung betrug 25,0 Gew.-% NaCl. Aus der Anodenkammer konnte eine 18,8 Gew.-%ige NaCl-Lösung entnommen werden. Der aus der Anodenkammer entnommenen NaCl-Lösung wurden 0,147kg/h an 19,5 Gew.%-igem angereicherten Gesamtprozessabwasser aus der osmotische Membrandestillation unter b) und 0,0594 kg/h festes Natriumchlorid zugegeben. Die Lösung wurde anschließend wieder in die Anoden-kammer eingespeist. Der Wassertransport über die Membran betrug 3,8 mol Wasser je mol Natrium.

Auf der Kathodenseite wurde eine Natronlauge mit einem Massenstrom von 0,653 kg/h umgepumpt. Die Konzentration der in die Kathodenseite eingespeisten Natronlauge betrug 30,0 Gew.-% NaOH, die aus der Kathodenseite entnommene Natronlauge hatte eine Konzentration von 32,7 Gew.-% NaOH. 0,182 kg/h der 32,7 %-igen Lauge wurden dem Volumenstrom entnommen, der Rest wurde mit 0,0539 kg/h Wasser aufgestockt und wieder in das Kathodenelement zurückgeführt.

32,7% des umgesetzten Natriumchlorids stammten aus dem DPC-Gesamtprozessabwasser.

### d) Rückführung der verdünnten Natriumhydroxid-lösung aus der osmotischen Destillation in die DPC-Herstellung

Die verdünnte Natriumhydroxid-Lösung aus b) wurde der Diphenylcarbonatherstellung zugeführt. Ein Gemisch aus 145,2 kg/h 14,5 %-iger Natronlauge, hergestellt durch Verdünnung von 105,0 kg/h der 20,0 %-igen Natronlauge aus b) mit 40,0 kg/h vollentsalztem Wasser (VE-Wasser), und 48,3 kg/h Phenol wurde mit einer Lösung von 86,2 kg/h Methylenchlorid und 27,5 kg/h Phosgen zusammengebracht und verfahren wie in 1a) beschrieben.

Durch die Verwendung der 20,0 %-igen statt der üblichen 32,0 %-igen Natriumhydroxid-Lösung konnte 39,4 kg/h (49,6 %) VE-Wasser eingespart werden.

### Vergleichsbeispiel 4 (Nachstellung Beispiel 1 aus DE 102006041465 A1)

### a) Isolierung des Reaktionsabwassers aus der DPC-Herstellung

Das Abwasser entsprach der Qualität gemäß Beispiel 1a. Das Reaktionsabwasser wurde nicht mit den Waschphasen vereinigt und wurde durch Strippen mit Wasserdampf von Lösungsmittelresten und Katalysator befreit. Nach Neutralisierung mit Salzsäure und Behandlung mit Aktivkohle enthielt das Reaktionsabwasser 17,0 Gew.-% NaCl und weniger als 2 ppm Phenol.

Das Abwasser konnte ohne weitere Reinigung der Natriumchlorid-Elektrolysezelle zugeführt werden.

### b) Elektrochemische Oxidation des Reaktionsabwassers aus a)

Die Elektrolyse wurde in einer Laborelektrolysezelle mit einer Anodenfläche von 0,01m² durchgeführt. Die Stromdichte betrug 4 kA/m², Temperatur Auslauf Kathodenseite 88°C, Temperatur Auslauf Anodenseite 89°C. Eingesetzt wurde eine Elektrolysezelle mit Standard Anoden- und Kathodencoating der Fa. DENORA, Deutschland. Eingesetzt wurde eine Ionenaustauschermembran der Fa. DuPont Nafion 982 WX. Die Elektrolysespannung betrug 3,02 V. Durch die Anodenkammer wurde eine Natriumchlorid-haltige Lösung mit einem Massenstrom von 0,8 kg/h umgepumpt. Die Konzentration der der Anodenkammer zugeführten Lösung betrug 25,0 Gew.-% NaCl. Aus der Anodenkammer konnte eine 18,6 Gew.-%ige NaCl-Lösung entnommen werden. Der aus der Anodenkammer entnommenen NaCl-Lösung wurden 0,133 kg/h an 17,0 Gew.-%igen Reaktionsabwasser aus der Diphenylcarbonatherstellung aus Beispiel 1a) und 0,0655 kg/h festes Natriumchlorid zugegeben. Die Lösung wurde anschließend wieder in die Anodenkammer eingespeist. Der Wassertransport über die Membran betrug 3,5 mol Wasser je mol Natrium.

Auf der Kathodenseite wurde eine Natronlauge mit einem Massenstrom von 0,653 kg/h umgepumpt. Die Konzentration der in die Kathodenseite eingespeisten Natronlauge betrug 30,0 Gew.-% NaOH, die aus der Kathodenseite entnommene Natronlauge hatte eine Konzentration von 33,0 Gew.-% NaOH. 0,180 kg/h der 33,0 %igen Lauge wurden dem Volumenstrom entnommen, der Rest wurde mit 0,060 kg/h Wasser aufgestockt und wieder in das Kathodenelement zurückgeführt.

Lediglich 25,8 % des umgesetzten Natriumchlorids stammten aus dem DPC-Reaktionsabwasser.

### Vergleichsbeispiel 5

### a) Isolierung des Gesamtprozessabwassers aus der DPC-Herstellung

Das Abwasser entsprach der Qualität gemäß Beispiel 2a. Das Reaktionsabwasser wurde mit den Waschphasen vereinigt und wurde durch Strippen mit Wasserdampf von Lösungsmittelresten und Katalysator befreit. Nach Neutralisierung mit Salzsäure und Behandlung mit Aktivkohle enthielt das Gesamtprozessabwasser 13,0 Gew.% NaCl und weniger als 2 ppm Phenol.

Das Abwasser konnte ohne weitere Reinigung der Natriumchlorid-Elektrolysezelle zugeführt werden.

### b) Elektrochemische Oxidation des Gesamtprozessabwassers

Die Elektrolyse wurde in einer Laborelektrolysezelle mit einer Anodenfläche von 0,01m² durchgeführt. Die Stromdichte betrug 4 kA/m², Temperatur Auslauf Kathodenseite 88°C, Temperatur Auslauf Anodenseite 89°C. Eingesetzt wurde eine Elektrolysezelle mit Standard Anoden- und Kathodencoating der Fa. DENORA, Deutschland. Eingesetzt wurde eine Ionenaustauschermembran der Fa. DuPont Nafion 982 WX. Die Elektrolysespannung betrug 3,02 V. Durch die Anodenkammer wurde eine Natriumchlorid-haltige Lösung mit einem Massenstrom von 0,8 kg/h umgepumpt. Die Konzentration der der Anodenkammer zugeführten Lösung betrug 25,0 Gew.-% NaCl. Aus der Anodenkammer konnte eine 18,6 Gew.-%ige NaCl-Lösung entnom-men werden. Der aus der Anodenkammer entnommenen NaCl-Lösung wurden 0,127 kg/h an 13,0 Gew.-%-igen Reaktionsabwasser aus der Diphenylcarbonatherstellung aus Beispiel 1a) und 0,0717 kg/h festes Natriumchlorid zugegeben. Die Lösung wurde anschließend wieder in die Anodenkammer eingespeist. Der Wassertransport über die Membran betrug 3,5 mol Wasser je mol Natrium.

Auf der Kathodenseite wurde eine Natronlauge mit einem Massenstrom von 0,653 kg/h umgepumpt. Die Konzentration der in die Kathodenseite eingespeisten Natronlauge betrug 30,0 Gew.-% NaOH, die aus der Kathodenseite entnommene Natronlauge hatte eine Konzentration von 33,0 Gew.-% NaOH. 0,180 kg/h der 33,0 %-igen Lauge wurden dem Volumenstrom entnommen, der Rest wurde mit 0,060 kg/h Wasser aufgestockt und wieder in das Kathodenelement zurückgeführt.

Lediglich 18,8 % des umgesetzten Natriumchlorids stammten aus dem DPC-Gesamtprozessabwasser

### Vergleichsbeispiel 6 (Nachstellung Beispiel 2 aus DE 102006041465 A1)

### a) Isolierung des Reaktionsabwassers aus der DPC-Herstellung

Das Abwasser entsprach der Qualität des Abwassers gemäß Beispiel 1a.

### b) Elektrochemische Oxidation des Reaktionsabwassers mit Gasdiffusionselektroden

Da für die Herstellung von DPC kein Wasserstoff benötigt wird, kann auf die Bildung von Wasserstoff bei der Elektrolyse verzichtet werden. Die Elektrolyse wurde daher mit Gasdiffusionselektroden betrieben. Die Stromdichte betrug 4 kA/m², Temperatur Auslauf Kathodenseite 88°C, Temperatur Auslauf Anodenseite 89 °C. Eingesetzt wurde eine Elektrolysezelle mit Standard Anoden-Coating der Fa. DENORA, Deutschland. Eingesetzt wurde eine Ionenaustauschermembran der Fa.DuPont, Nafion 982 WX. Die Elektrolysespannung betrug 2,11 V. Die Natriumchlorid-Konzentration der der Anodenkammer entnommenen Lösung betrug 17,0 Gew.% an NaCl. Der aus der Anodenkammer entnommenen NaCl-Lösung wurden 0,178 kg/h an 17,0 Gew.%-igem Reaktionsabwasser und 0,0579 kg/h festes Natriumchlorid zugegeben. Die Lösung wurde anschließend wieder in die Anodenkammer eingespeist. Der Wassertransport über die Membran betrug 4,9 mol Wasser je mol Natrium.

Auf der Kathodenseite wurde eine Natronlauge mit einem Massenstrom von 0,653 kg/h umgepumpt. Die Konzentration der in die Kathodenseite eingespeisten Natronlauge betrug 30,0 Gew.% NaOH, die aus der Kathodenseite entnommen Natronlauge hatte eine Konzentration von 31,5 Gew.% NaOH. 0,189 kg/h der 31,5 %-igen Lauge wurden dem Volumenstrom entnom-men, der Rest wurde mit 0,0312 kg/h Wasser aufgestockt und wieder in das Kathodenelement zurückgeführt. Der Anteil an umgesetztem Natriumchlorid aus dem DPC-Reaktionsabwasser betrug 34,4 %.

Die Beispiele zeigen, dass eine wesentlich höhere Recyclierungsquote des Natriumchlorids in den Abwasserlösungen des DPC-Herstellungsprozess bei der Elektrolyse nach Aufkonzentrierung durch osmotische Membrandestillation erreicht wird, wobei das von der Alkalilauge aufgenommene Wasser bei der Herstellung der Alkalilaugelösung im DPC-Herstellungsschritt eingespart werden kann.

## Patentansprüche

1. Verfahren zur Herstellung von Diarylcarbonate umfassend folgenden Schritte:
a) Herstellung von Phosgen durch Umsetzung von Chlor mit Kohlenmonoxid,
b) Umsetzung des gemäß Schritt a) gebildeten Phosgens mit wenigstens einem Monophenol in Gegenwart einer wässrigen Alkali-haltigen Base, insbesondere einer Natrium-haltigen Base, gegebenenfalls eines Stickstoffkatalysators, zu einem Diarylcarbonat und einer Alkalichlorid-haltigen, insbesondere einer Natriumchlorid-haltigen Reaktionsabwasserlösung,
c) Abtrennung und Aufarbeitung des in Schritt b) gebildeten Diarylcarbonats,
e) Aufkonzentrierung wenigstens eines Teils der gemäß Schritt c) verbliebenen Alkalichlorid-haltigen Lösung durch osmotische Membrandestillation,
f) Elektrochemische Oxidation wenigstens eines Teils der Alkalichlorid-haltigen Lösung aus e) unter Bildung von Chlor, Alkalilauge und gegebenenfalls Wasserstoff.

2. Verfahren gemäß Anspruch 1, **dadurch gekennzeichnet, dass** in einem Schritt d) eine Abtrennung der Lösungsmittelreste und gegebenenfalls Katalysatorreste von der gemäß Schritt c) verbliebenen Alkalichlorid-haltigen Lösung, insbesondere durch Extraktion oder Strippen der Lösung mit Wasserdampf, und/oder Behandlung mit Adsorbentien, insbesondere mit Aktivkohle und anschließend der osmotischen Membrandestillation im Schritt e) zugeführt wird.

3. Verfahren gemäß Anspruch 1 oder 2, **dadurch gekennzeichnet, dass** die Alkalichlorid-haltige Lösung aus d) vor der Elektrolyse f) mittels osmotische Destillation im Schritt e) mit Natronlauge als Wasserakzeptor aufkonzentriert wird.

4. Verfahren gemäß einem der voranstehenden Ansprüche, **dadurch gekennzeichnet, dass** die osmotische Mem-brandestillation e) bei einer Temperatur von 20 bis 50°C betrieben wird,

5. Verfahren gemäß einem der voranstehenden Ansprüche, **dadurch gekennzeichnet, dass** die osmotische Membrandestillation e) bei einem Absolutdruck von 110 kPa bis 120 kPa (1,1 bis 1,2 bar) betrieben wird.

6. Verfahren gemäß einem der voranstehenden Ansprüche, **dadurch gekennzeichnet, dass** die osmotische Membrandestillation e) mit einem Differenzdruck von 3 MPa bis 10 MPa (30 bis 100 bar) betrieben wird.

7. Verfahren gemäß einem der voranstehenden Ansprüche, **dadurch gekennzeichnet, dass** die osmotische Membrandestillation wenigstens eines Teils der Alkalichlorid-haltigen Lösung aus d) mit einer lipophilen Membran erfolgt.

8. Verfahren gemäß einem der voranstehenden Ansprüche, **dadurch gekennzeichnet, dass** die elektrochemische Oxidation wenigstens eines Teiles der Alkalichlorid-haltigen Lösung aus e) zu Chlor, und Natronlauge unter Einsatz von Gasdiffusionselektroden als Kathode erfolgt.

9. Verfahren gemäß einem der voranstehenden Ansprüche, **dadurch gekennzeichnet, dass** wenigstens ein Teil der gereinigten Alkalichlorid-haltigen Lösung aus e) in den Solekreislauf einer Membranelektrolyse zur Herstellung von Chlor, Natronlauge gegeben wird.

10. Verfahren gemäß einem der voranstehenden Ansprüche, **dadurch gekennzeichnet, dass** der Alkalichlorid-haltigen Lösung bei der Elektrolyse f) zusätzliches Alkalichlorid zur Erhöhung der Alkalichlorid-Konzentration zugegeben wird.

11. Verfahren gemäß einem der voranstehenden Ansprüche, **dadurch gekennzeichnet, dass** die Alkalichlorid-haltige Lösung bei der Abtrennung in Schritt c) oder der Reinigung in Schritt d) auf einen pH-Wert von kleiner als 8 eingestellt wird.

12. Verfahren gemäß Anspruch 11, **dadurch gekennzeichnet, dass** der pH-Wert der Alkalichlorid-haltigen Lösung bei der Abtrennung in Schritt c) oder der Reinigung in Schritt d) durch Einsatz von Salzsäure oder Chlorwasserstoff eingestellt wird.

13. Verfahren gemäß einem der voranstehenden Ansprüche, **dadurch gekennzeichnet, dass** die Alkalichlorid-Konzentration, der in die Elektrolyse gelangenden Alkalichlorid-Lösung von 100 bis 280 g/l, bevorzugt 110 bis 220 g/l beträgt und/oder dass die Konzentration der Natronlauge, die aus der Elektrolyse erhalten wird, 13 bis 50 Gew.-%, bevorzugt 14 bis 32 Gew.-%, beträgt.

14. Verfahren gemäß einem der voranstehenden Ansprüchen, **dadurch gekennzeichnet, dass** Phenole der Formel (I) 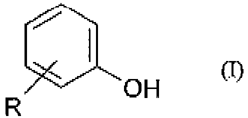 worin R Wasserstoff, Halogen oder ein verzweigter unverzweigter C₁ bis C₉-Alkylrest oder Alkoxycarbonylrest ist, in der Reaktion b) eingesetzt werden.

15. Verfahren gemäß einem der voranstehenden Ansprüchen, **dadurch gekennzeichnet, dass** als Monophenol in Stufe b) Phenol, Alkylphenol, insbesondere Kresole, p-tert.-Butylphenol, p-Cumylphenol, p-n-Octylphenol, p-iso-Octylphenol, p-n-Nonylphenol und p-iso-Nonyl-phenol, Halogenphenole insbesondere p-Chlorphenol, 2,4-Dichlorphenol, p-Bromphenol und 2,4,6-Tribromphenol, besonders bevorzugt Phenol eingesetzt wird.

## Claims

1. Process for preparing diaryl carbonate, which comprises the following steps:
a) preparation of phosgene by reaction of chlorine with carbon monoxide,
b) reaction of the phosgene formed in step a) with at least one monophenol in the presence of an aqueous alkali metal-containing base, in particular a sodium-containing base, optionally a nitrogen catalyst, to form a diaryl carbonate and a reaction wastewater solution containing alkali metal chloride, in particular sodium chloride,
c) separation and work-up of the diaryl carbonate formed in step b),
e) concentration of at least part of the solution containing alkali metal chloride which remains after step c) by osmotic membrane distillation,
f) electrochemical oxidation of at least part of the solution containing alkali metal chloride from e) to form chlorine, alkali metal hydroxide and if appropriate hydrogen.

2. Process according to Claim 1, **characterized in that**, in a step d), a separation of the solvent residues and optionally catalyst residues from the solution containing alkali metal chloride which remains after step c), in particular by extraction or stripping of the solution by means of steam and/or treatment with adsorbents, in particular activated carbon, is carried out and the solution is subsequently fed to the osmotic membrane distillation in step e).

3. Process according to Claim 1 or 2, **characterized in that** the solution containing alkali metal chloride from d) is concentrated by means of osmotic distillation in step e) using sodium hydroxide solution as water acceptor prior to the electrolysis f).

4. Process according to any of the preceding claims, **characterized in that** the osmotic membrane distillation e) is carried out at a temperature of from 20 to 50°C.

5. Process according to any of the preceding claims, **characterized in that** the osmotic membrane distillation e) is carried out at an absolute pressure of from 110 kPa to 120 kPa (1.1 to 1.2 bar).

6. Process according to any of the preceding claims, **characterized in that** the osmotic membrane distillation e) is carried out at a differential pressure of from 3 MPa pt 10 MPa (30 to 100 bar).

7. Process according to any of the preceding claims, **characterized in that** the osmotic membrane distillation of at least part of the solution containing alkali metal chloride from d) is carried out using a lipophilic membrane.

8. Process according to any of the preceding claims, **characterized in that** the electrochemical oxidation of at least part of the solution containing alkali metal chloride from e) to chlorine and sodium hydroxide is carried out using gas diffusion electrodes as cathode.

9. Process according to any of the preceding claims, **characterized in that** at least part of the purified solution containing alkali metal chloride from e) is introduced into the brine circuit of a membrane electrolysis for the preparation of chlorine, sodium hydroxide.

10. Process according to any of the preceding claims, **characterized in that** additional alkali metal chloride is added to the solution containing alkali metal chloride to increase the alkali metal chloride concentration in the electrolysis f).

11. Process according to any of the preceding claims, **characterized in that** the solution containing alkali metal chloride is brought to a pH of less than 8 in the isolation in step c) or the purification in step d).

12. Process according to Claim 11, **characterized in that** the pH of the solution containing alkali metal chloride is adjusted by use of hydrochloric acid or hydrogen chloride in the isolation in step c) or the purification in step d).

13. Process according to any of the preceding claims, **characterized in that** the alkali metal chloride concentration of the alkali metal chloride solution fed to the electrolysis is from 100 to 280 g/l, preferably from 110 to 220 g/l, and/or **in that** the concentration of the sodium chloride solution obtained from the electrolysis is from 13 to 50% by weight, preferably from 14 to 32% by weight.

14. Process according to any of the preceding claims, **characterized in that** phenols of the formula (I) 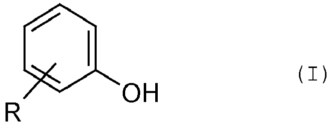 where R is hydrogen, halogen or a branched or unbranched C₁-Cg-alkyl radical or alkoxycarbonyl radical, are used in the reaction b).

15. Process according to any of the preceding claims, **characterized in that** phenol, alkylphenols, in particular cresols, p-tert-butylphenol, p-cumylphenol, p-n-octylphenol, p-isooctylphenol, p-n-nonylphenol and p-isononylphenol, halophenols in particular p-chlorophenol, 2,4-dichlorophenol, p-bromophenol and 2,4,6-tribromophenol, particularly preferably phenol, is used as monophenol in step b).

## Revendications

1. Procédé de fabrication de carbonate de diaryle comprenant les étapes suivantes :
a) la fabrication de phosgène par mise en réaction de chlore avec du monoxyde de carbone,
b) la mise en réaction du phosgène formé selon l'étape a) avec au moins un monophénol en présence d'une base aqueuse contenant un alcali, notamment d'une base contenant du sodium, éventuellement d'un catalyseur azoté, pour former un carbonate de diaryle et une solution d'eau résiduaire de réaction contenant un chlorure alcalin, notamment du chlorure de sodium,
c) la séparation et le traitement du carbonate de diaryle formé à l'étape b),
e) la concentration d'au moins une partie de la solution contenant un chlorure alcalin restante selon l'étape c) par distillation sur membrane osmotique,
f) l'oxydation électrochimique d'au moins une partie de la solution contenant un chlorure alcalin de e) avec formation de chlore, d'une lessive alcaline et éventuellement d'hydrogène.

2. Procédé selon la revendication 1, **caractérisé en ce que**, dans une étape d), une séparation des résidus de solvant et éventuellement des résidus de catalyseur de la solution contenant un chlorure alcalin restante selon l'étape c) a lieu, notamment par extraction ou stripage de la solution avec de la vapeur d'eau et/ou traitement avec des adsorbants, notamment avec du charbon actif, puis celle-ci est introduite dans la distillation sur membrane osmotique à l'étape e).

3. Procédé selon la revendication 1 ou 2, **caractérisé en ce que** la solution contenant un chlorure alcalin de d) est concentrée avant l'électrolyse f) par distillation osmotique à l'étape e) avec de la soude caustique en tant qu'accepteur d'eau.

4. Procédé selon l'une quelconque des revendications précédentes, **caractérisé en ce que** la distillation sur membrane osmotique e) est réalisée à une température de 20 à 50 °C.

5. Procédé selon l'une quelconque des revendications précédentes, **caractérisé en ce que** la distillation sur membrane osmotique e) est réalisée à une pression absolue de 110 kPa à 120 kPa (1,1 à 1,2 bar).

6. Procédé selon l'une quelconque des revendications précédentes, **caractérisé en ce que** la distillation sur membrane osmotique e) est réalisée à une pression différentielle de 3 MPa à 10 MPa (30 à 100 bar).

7. Procédé selon l'une quelconque des revendications précédentes, **caractérisé en ce que** la distillation sur membrane osmotique d'au moins une partie de la solution contenant un chlorure alcalin de d) a lieu avec une membrane lipophile.

8. Procédé selon l'une quelconque des revendications précédentes, **caractérisé en ce que** l'oxydation électrochimique d'au moins une partie de la solution contenant un chlorure alcalin de e) en chlore et soude caustique a lieu en utilisant des électrodes à diffusion de gaz en tant que cathode.

9. Procédé selon l'une quelconque des revendications précédentes, **caractérisé en ce qu'**au moins une partie de la solution contenant un chlorure alcalin purifiée de e) est ajoutée dans le circuit de saumure d'une électrolyse sur membrane pour la fabrication de chlore, de soude caustique.

10. Procédé selon l'une quelconque des revendications précédentes, **caractérisé en ce qu'**un chlorure alcalin supplémentaire est ajouté à la solution contenant un chlorure alcalin lors de l'électrolyse f) pour augmenter la concentration en chlorure alcalin.

11. Procédé selon l'une quelconque des revendications précédentes, **caractérisé en ce que** la solution contenant un chlorure alcalin est ajustée à un pH inférieur à 8 lors de la séparation à l'étape c) ou de la purification à l'étape d).

12. Procédé selon la revendication 11, **caractérisé en ce que** le pH de la solution contenant un chlorure alcalin est ajusté lors de la séparation à l'étape c) ou de la purification à l'étape d) en utilisant de l'acide chlorhydrique ou du chlorure d'hydrogène.

13. Procédé selon l'une quelconque des revendications précédentes, **caractérisé en ce que** la concentration en chlorure alcalin de la solution de chlorure alcalin atteignant l'électrolyse est de 100 à 280 g/l, de préférence de 110 à 220 g/l, et/ou **en ce que** la concentration de la soude caustique qui est obtenue lors de l'électrolyse est de 13 à 50 % en poids, de préférence de 14 à 32 % en poids.

14. Procédé selon l'une quelconque des revendications précédentes, **caractérisé en ce que** des phénols de formule (I) 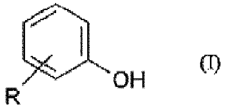 dans laquelle R est l'hydrogène, un halogène ou un radical alkyle en C₁ à C₉ ramifié ou non ramifié ou un radical alcoxycarbonyle, sont utilisés dans la réaction b).

15. Procédé selon l'une quelconque des revendications précédentes, **caractérisé en ce que** du phénol, des alkylphénols, notamment des crésols, du p-tert.-butylphénol, du p-cumylphénol, du p-n-octylphénol, du p-iso-octylphénol, du p-n-nonylphénol et du p-iso-nonyl-phénol, des halogénophénols, notamment du p-chlorophénol, du 2,4-dichlorophénol, du p-bromophénol et du 2,4,6-tribromophénol, de manière particulièrement préférée du phénol, sont utilisés en tant que monophénol à l'étape b).
